# EUROPÄISCHE PATENTANMELDUNG

(11) **EP 1 840 569 A1**
(43) Veröffentlichungstag der Anmeldung: **03.10.2007**
(21) Anmeldenummer: 06090038.8
(22) Anmeldetag: 28.03.2006
(51) Int. Cl.: G01N 33/50, G01N 33/53, G01N 33/537

(54) **Verwendung des 4-1BB Rezeptors zur Identifizierung und/oder Separation aktivierter regulatorischer Th-Zellen (Treg)**

(71) Anmelder: Deutsches Rheuma-Forschungszentrum Berlin, 10117 Berlin (DE)
(72) Erfinder: Thiel, Andreas, 10965 Berlin (DE); Schönbrunn, Anne, 13055 Berlin (DE); Frentsch, Marco, 12161 Berlin (DE)
(74) Vertreter: Boeckh, Tobias

(57) **Zusammenfassung**

Es wird die Verwendung des 4-1BB Rezeptors zur Identifizierung und/oder Separation von, spezifischen regulatorischen Th-Zellen nach Aktivierung mit Antigen, polyklonalen regulatorischen Th-Zellen nach polyklonaler Aktivierung und ein Verfahren zur Identifizierung und/oder Separation von regulatorischen Th-Zellen vorgeschlagen, wobei die regulatorischen T-Zellen anhand der Ausprägung des 4-1BB Rezeptors durch einen Antikörper, Antigen oder Liganden gekoppelt mit einem fluoreszierenden Stoff, Haptenen oder magnetischen Mikropartikeln detektiert werden und ein Kit, umfassend einen Antikörper, Antigen oder Liganden zur Detektion des 4-1BB Rezeptors zur Identifikation und/oder Separation einer regulatorischen Th-Zelle, wobei der Antikörper mit einem fluoreszierenden Stoff, Haptenen oder magnetischen Mikropartikeln gekoppelt ist, vorgeschlagen.

## Beschreibung

Die vorliegende Erfindung betrifft die Verwendung eines 4-1BB Rezeptors zur Identifizierung und/oder Separation von aktivierten regulatorischen T-Zellen und einen Kit, umfassend einen Antikörper zur Detektion des 4-1BB Rezeptors (CD137) zur Identifikation und/oder Separation aktivierter regulatorischer Th-Zellen, wobei der Antikörper an einen fluoreszierenden Stoff oder magnetische Mikropartikel gekoppelt ist. Die regulatorischen T Zellen können hierbei sowohl polyklonal als auch mit spezifischen Antigenen aktiviert sein. Die Aktivierung kann in vitro oder bereits in vivo erfolgt sein.

Im Organismus existieren spezifische T-Zellen, die nicht erwünschte Immunreaktionen wie zum Beispiel gegenüber körpereigenen Strukturen (Autoantigenen) aktiv unterdrücken. Sie werden als regulatorische T-Zellen (Treg) bezeichnet. Regulatorische T-Zellen sind essentiell and der lebenslangen Erhaltung der peripheren Toleranz gegenüber Autoantigenen beteiligt. Sie werden im Thymus gebildet, dessen Aktivität jedoch mit zunehmendem Alter stark reduziert ist.

Regulatorische T-Zellen können zur Unterdrückung von Anti-Tumor-Antworten führen (Onizuka et al., Cancer Res. 59: 3128-3133 (1999); Shimizu et al., J. Immunol. 163:5211-5218 (1999)), da viele Tumorantigene klassische Autoantigene repräsentieren. Dagegen können verminderte Anzahlen von Treg oder funktionale Veränderungen von Treg zu Autoimmunkrankheiten führen, in deren Verlauf dann körpereigene Strukturen wie Fremdstoffe oder Pathogene unkontrolliert attackiert werden.
Tregs sind an der Aufrechterhaltung der immunologischen Selbsttoleranz beteiligt, indem sie die Aktivierung autoreaktive T-Zellen unterbinden. Sie sind in der Lage, sowohl die Cytokinproduktion als auch die Proliferation solcher potentiell pathogener T-Zellen zu supprimieren. Ein wesentlicher Schritt zur Identifizierung von regulatorischen T-Helferzellen war die Charakterisierung von CD4+ T-Helferzellen, die die α-Kette des Interleukin-2 (IL-2) Rezeptors (CD25) konstitutiv als Oberflächenmembranprotein aufwiesen. Die funktionelle Bedeutung und die genauen molekularen Mechanismen der Suppression dieser CD25+ CD4+ regulatorischen T-Zellen und auch wie sie entstehen, sind bisher nicht geklärt. Da der CD25 Rezeptor auch von Subpopulationen nicht-regulatorischer T-Zellen ausgeprägt wird, kann dieser Marker nur bedingt zur Analyse und Anreicherung von Treg verwendet werden. Insbesondere aber kann CD25 nicht zur Identifizierung und Separation von aktivierten, Antigen-spezifischen regulatorischen T Zellen verwendet werden.

Eine Isolierung von Treg und im besonderen von Treg spezifisch für bestimmte Antigene ist dennoch eines der grossen therapeutischen Ziele. Ebenso die Analyse von Antigenspezifischen Treg! (Auto)-Antigen-spezifische Tregs sind ein spezifisches Mittel zur Unterdrückung von ungewollten Immunreaktionen wie z.B. Autoimmunreaktionen bei Rheumatoider-Arthritis (RA), Multipler Sklerose (MS), Atherosklerose (AS), Diabetes, Psoriasis. Andere ungewollte Immunreaktionen bei denen Treg ein spezifsches Mittel darstellen würden sind die GvHD (graft vesus host disease) bei allogene Stamzelltransplantationen oder die Transplantatabstoßung bei Organtransplantationen (Hara et al., J. Immunol. 166: 3789-3796(2001); Taylor et al., J. Exp. Med. 193: 1311-1318 (2001). Auch Allergien stellen ungewollte Immunreaktionen dar, für die es bisher nur wenige therapeutische Optionen gab. Eine therapeutische Behandlung mit Treg, die auf dem natürlichen Prinzip von peripherer Toleranz beruht und in vielen experimentellen Modellen bewiesen ist, würde keine Nebenwirkungen im Vergleich zu klassischen immunosuppressiven Medikamenten beinhalten und wäre kurativ.

Hinsichtlich der aufgeführten Bedeutung der Tregs sind die an der Supression beteiligten Moleküle und Mechanismen sowie zuverlässige Treg-Marker von großer Bedeutung. Im Stand der Technik ist ein molekularbiologischer Treg-Marker, der zur Forkhead-Familie gehörende Transkriptionsrepressor FoxP3, anerkannt (Hori et al (2003) Science 99(5609):1057-61). Des weiteren können regulatorische T-Zellen anhand der Expression des CD25 Moleküls identifiziert werden (Thornton and Shevach (1998) J. Exp. Med. 188 287-296; Sakaguchi et al (1995) J.Immunol. 155 1151-1164).

Choi et al 2004, JLB, offenbaren, dass bisher keine Anhaltspunkte existieren, dass 4-1BB als diskriminativer Marker für Treg gegenüber CD25 negativen Zellen einsetzbar ist.

Allerdings ist es bisher vor allem nicht möglich, die Treg zu identifizieren und zu isolieren, die ein spezifisches Antigen erkennen. Dies wäre möglich, wenn spezifisch aktivierte Treg separiert werden könnten und wäre eine Grundvoraussetzung für spezifische Therapien mit Treg zum Beispiel bei Autoimmunerkrankungen, in denen die der Krankheit zu Grunde liegenden Autoimmunreaktivitäten unterdrückt werden sollten nicht aber Immunreaktivitäten gegen Tumorzellen.

Es ist somit schwierig nach dem bisherigen Stand der Technik regulatorische T-Zellen zu identifizieren und/oder zu isolieren. Bisherige oben beschriebene Marker lassen nicht zu, dass die Gesamtheit der regulatorischen T-Zellen identifiziert werden kann, da nicht alle regulatorischen T-Zellen definiert spezifische Marker exprimieren. Demzufolge können bisher nur Subpopulationen identifiziert und/oder separiert werden wie zum Beispiel nur die Treg, die den CD25 Rezeptor stark ausprägen. Des Weiteren können verschiedene andere Zellsubpopulationen die gleichen Zelloberflächenmarker besitzen (wie CD4 für Th1 Th2, oder anderen Th-Zellen oder CD25 für aktivierte T-Zellen oder B-Zellen.
Insbesondere ist jedoch die Identifizierung aktivierter Treg nach Stimulation mit definierten Antigenen anhand spezifischer Aktivierungsmarker nicht möglich. Die Aktivierung von regulatorischen Th-Zellen führt zur zwar zur verstärkten Expression von CD25 und CD38, die aber wie alle anderen beschriebenen T-Zellaktivierungsmarker auch in anderen T-Zellen exprimiert werden, so dass bisher keine Antigenspezifischen Treg anhand spezifischer Aktivierungsmarker separiert werden können.

Die Mehrzahl der Methoden, die anhand der Expression eines bestimmten Zelloberflächenmarkers regulatorische Th-Zellen identifizieren und/oder isolieren hängen von der Erkennung eines Markers und der Bindung eines Antikörpers ab. Wenn nicht ein einziger Marker verwendet werden kann, um einen bestimmten Zelltyp zu identifizieren und/oder zu isolieren, muss eine Kombination von Markern und den zu ihnen passenden Antikörpern gefunden werden (Levingset al., J Exp. Med. 193 (11): 1295-1302 (2001)). Solche Experimente können in der Praxis sehr komplex und schwierig in der Durchführung sein. Des Weiteren kann die Bindung der Antikörper die Aktivität der Zielzelle oder die Expression der anderen Marker beeinflussen, so dass der Identifikation und/oder Separationsprozess mit den weiteren Antikörpern negativ beeinflusst wird.

Abschließend soll noch angemerkt werden, dass es durch die Bindung von Antikörpern zu einer Querverbindung (crosslinking), Kappung und Internalisation der betreffenden Marker kommen kann. Hierdurch kann die Zellfunktion eingeschränkt werden. Auch wenn Fab-Fragmente verwendet werden, sind die betreffenden Marker maskiert und können unter Umständen in ihrer Funktion beeinträchtigt sein. Die Bindung an bestimmte Marker kann auch zur Deaktivierung der Zelle führen (CD28, go3), Apoptose induzieren (Quervernetzung von CD4 in aktivierten Zellen) oder die Sekretion von Zytokinen induzieren (CD3, CD2, CD28).
Anhand des Standes der Technik konnte bisher keine spezifische Identifizierung und/oder Separation von ausschließlich lebenden regulatorischen T-Zellen durchgeführt werden. Der einzig bisher beschriebene spezifische Marker FoxP3 kann zudem neueren Untersuchungen folgend auch in nichtregulatorischen T-Zellen induziert werden (Fontenot J.D. 2003, Hori S. et al. 2003). Da FoxP3 als intrazelluläres Protein vorliegt, ist es darüberhinaus nach dem bisherigen Stand der Technik nicht möglich, lebende regulatorische Zellen zum Beispiel anhand eines FoxP3-Antikörpers zu identifizieren und/oder zu isolieren. Wiederum stellt sich zusätzlich das Problem, dass nur die Gesamheit der Treg identifiziert werden kann, nicht aber Treg spezifisch für zuvor definierte Antigene. Daher ist eine zuverlässige Identifikation und/oder Separation von Treg und inbesondere Antigen-spezifischer Treg bisher nicht möglich.

Es wurde überraschend ein Verfahren gefunden, wodurch man lebende, aktivierte regulatorische Th-Zellen (Treg) identifizieren und/oder isolieren kann, wobei man den 4-1BB-Rezeptor als Marker und 4-1BB-spezifische Antikörper zur Identifikation und/oder Isolierung der aktivierten, regulatorische T-Zellen verwendet.

Die regulatorischen Zellen können durch die Expression eines und/oder mehrerer Marker identifiziert und/oder separiert werden. Hierfür können alle dem Fachmann bekannten Marker zur Identifizierung und/oder Separation eingesetzt werden. Bevorzugt werden die Marker 4-1BB, CD25, CTLA-4 (cytotoxic T lymphocyte antigen-4), GITR (Glucocorticoidinduced TNFRezeptor), FoxP3, IL-10, CD69, CD40L, ICOS, OX40 und TGFβ, die einzeln und/oder in Kombination eingesetzt werden. Hierfür können auch alle dem Fachmann bekannten Marker zum Ausschluß oder der Depletion von nichtregulatorischen Zellen in Kombination eingesetzt werden. Besonders bevorzugt ist aber die Verwendung des 4-1BB Rezeptors als Marker von lebenden, aktivierten regulatorischen Zellen. Entweder werden direkt aktivierte regulatorische T-Zellen identifiziert oder separiert oder die Aktivierung wird durch Zugabe von Zellen, Proteinen, Peptiden, Pathogenen oder anderen Stoffen induziert.

Die getrennten Zellen können in jedem entsprechenden Behältnis - z.B. einer Sammelröhre - aufgefangen und/oder gesammelt werden, welches das Überleben und/oder die Vermehrung der Zellen ermöglicht. Verschiedene Medien sind im Handel verfügbar und können je nach der Art der identifizierten und/oder separierten Zellen gebraucht werden. Diese Medien können z.B. dMEM, HBSS, dPBS, RPMI, Iscove's Medium usw. sein, welche häufig mit fötalem Kalbsserum, humanem Serum oder Serumersatzstoffen ergänzt werden.

Die identifizierten und/oder separierten Zellpopulationen können sofort benutzt werden oder nach Isolierung in vitro kultiviert werden. Hiernach können die Zellen eingefroren werden, oder man friert sie vor dem Trennungsverfahren ein. Falls die Zellen für einen längeren Zeitraum gelagert werden, ist es bevorzugt sie bei Temperaturen um -80°C oder in flüssigen Stickstoff einzufrieren, um zu garantieren, dass sie nach dem Auftauen wieder verwenden werden können. Hierbei werden die Zellen normalerweise in DMSO und/oder FCS/HS in Verbindung mit einem Medium, Glucose usw. gelagert. Sobald aufgetaut, können die Zellen entweder direkt für therapeutische Zwecke oder in vitro Versuche verwendet werden oder durch die Verwendung von Wachstumsfaktoren, Antigenen, Zellen usw. vermehrt und/oder differenziert werden.

Bevorzugt ist es, die regulatorischen Th-Zellen aus einem Zellgemisch zu identifizieren und zu isolieren. Die regulatorischen Th-Zellen werden aus Zellproben von Säugetieren (Mamalia), aber besonders von Menschen und bevorzugt aus gesunden Probanden und/oder Patienten identifiziert und/oder separiert. Die Zellproben können z.B. aus Blutproben, die Immunzellen enthalten (peritoneale, cerebrale-spinale, pleurale und/oder synoviale Körperflüssigkeit), Spülungsflüssigkeiten von Hohlorganen (Atemwege, Lungen), Homogenaten und Aspirata von Lymphknoten, Milz, Mandeln und/oder anderem lymphatischem Gewebe stammen. Die Zellen können aus Tiergeweben gewonnen werden (z.B. von der Milz oder den Lymphknoten eines Tieres). Die Zellen können auch Teil einer Blutprobe sein, z.B. von einer Versuchperson oder menschlichem Patienten, besonders bevorzugt aus einer peripheren Blutprobe.

In einer weiteren bevorzugten Ausführung, ist vorgesehen, dass aktivierte regulatorische Th-Zellen identifiziert und/oder separiert werden.

Aktivierte regulatorische Th-Zellen sind direkt in der Lage, die Aktivität von T-Zellen zu unterdrücken. In Tiermodellen ist gezeigt worden, dass aktivierte regulatorischen T-Zellen die Entwicklung von Autoimmunerkrankungen, von Transplantatabstoßungsreaktionen und allergischen Reaktionen verhindern können. In Analogie dazu vermutet man, dass aktivierte regulatorische T-Zellen auch beim Menschen die Entstehung von chronischen Immunreaktionen verhindern können. Durch die Verwendung des 4-1BB Markers werden aktivierte regulatorische Th-Zellen spezifisch identifiziert und/oder separiert. Bevorzugt werden zuvor in vitro aktivierte regulatorische Th-Zellen mit dem Phänotyp CD4+ CD25+ FoxP3+ identifiziert und/oder separiert werden. Die Aktivierung erfolgt hierbei bevorzugt mittels Antigenen, Antikörpern, Peptiden, Proteinen, chemischen Verbindungen (oder Gemischen solcher Stoffe) oder Pathogenen oder Zellen. Eine weitere besondere Ausführungsform der Erfindung ist es, lebende regulatorische Th-Zellen direkt ex vivo zu identifizieren und/oder zu isolieren.

Durch die Verwendung des 4-1BB Markers und dem entsprechenden 4-1BB Antigen können bevorzugt aktivierte regulatorischen Th-Zellen identifiziert und/oder separiert werden, was eine eine bevorzugte Verwendung der Erfindung, zur Indentifizierung und Isolierung Antigen-spezifischer regulatorische Th-Zellen erlaubt.

Nach der Gewinnung eines Zellgemisches aus einem Patienten und/oder Probanten, kann diese mit bestimmten Antigenen stimuliert werden. Hierzu können Antikörper, Peptide, Proteine, chemische Verbindungen (oder Gemische solcher Stoffe) oder Pathogene oder Zellen verwendet werden. Nach einer bestimmten Zeit der Aktivierung werden durch die Verwendung des 4-1BB Markers die regulatorischen Th-Zellen identifiziert und/oder separiert, Hierbei werden bevorzugt Antigen-spezifische regulatorische Th-Zellen identifiziert und/oder separiert werden.

Die Zellen die nach der Erfindung separiert werden stammen im Allgemeinen von einer in vivo Quelle ab und reflektieren daher den immunologischen Status des Spenders in Bezug auf die Anzahl, den Standort und die T Zellantigenrezeptorspezifität der Treg Zellen wieder. Diese Informationen können bei der diagnostischen Prüfungen verwendet werden, die sich auf immunologische Störungen beziehen, z.B. Krebs bezogene Immunosuppression, autoimmune Störungen; atopische Zustände, usw.

Des Weiteren können Th-Zellen des Zielpatienten in vitro Zellen, Zellextrakten, separierten Antigenen oder Proteinen des Organspenders vor oder nach der Transplantation ausgesetzt werden. Hiernach würden die aktiven regulatorischen Th-zellen nach einer angebrachten Aktivierungszeit anhand des 4-1BB Markers identifiziert und/oder separiert werden.

Die Zellen können durch alle dem Fachmann bekannten Techniken identifiziert und/oder separiert werden. Zur Identifikation der Zellen eignen sich bevorzugt die Methoden magnetische Zellsortierung (MACS), fluoreszenzaktivierte Zellsortierung (FACS), ELISA, PCR und/oder alle dem Fachmann bekannte Fluoreszenzmikroskope. Besonders bevorzugt eignet sich die Durchflußzytometrie (FACS). Für die Separation der Zellen eignen sich bevorzugt die Zelltrennungssysteme, durch die Zellen mit Hilfe eines Magnetfeldes (MACS) oder durch Durchflußzytrometrie (FACS) sortiert werden. Bevorzugt ist es, dass die Expression des 4-1BB Rezeptors nach einer Stimulation gemessen wird.

Die regulatorischen Th-Zellen werden durch dem Fachmann bekannte Stoffe stimuliert. Nach 1h bis 18h Stimulation wird die Expression des 4-1BB Rezeptors gemessen. Bevorzugt wird die Expression nach 3h bis 8h einer Stimulation gemessen.
Der bevorzugte optimale Stimulationszeitpunkt ist der Zeitpunkt an dem möglichst viele der aktivierten, regulatorischen T-Zellen, aber möglichst wenige der nichtregulatorischen Zellen 4-1B exprimieren. Dieser Zeitpunkt kann vom Fachmann durch eine Analyse der 4-1BB Expression zu verschiedenen Zeitpunkten nach Stimulation ermittelt werden.

In einer weiteren bevorzugten Ausführungsform, ist vorgesehen, dass die regulatorischen Th-Zellen nach einer Stimulation der Zellen im Zellgemisch identifiziert und/oder separiert werden.

Des Weiteren ist eine bevorzugte Verwendung der Erfindung, dass die Zellen durch Antigene, Proteine, Peptide, chemische Stoffe, Zellen, Wachstumsfaktoren, Antikörper und/oder Liganden stimuliert werden.

Die Kultur kann zudem geeignete Wachstumsfaktoren enthalten. Wachstumsfaktoren sind definiert als Moleküle, die das Überleben, Wachstum und/oder die Differenzierung einer Zelle entweder in Kultur oder im intakten Gewebe durch bestimmte Wirkungen auf einen transmembrane Rezeptor fördern. Wachstumsfaktoren schließen Polypeptide und nicht-polypeptide Faktoren ein. Bestimmte Wachstumsfaktoren, die verwendet werden können, die separierte und/oder verwendete Zelle zu kultivieren, schließen die Interleukine, z.B. IL -1, IL -2, IL -3, IL -4, IL -5, IL -6, IL -7, IL -8, IL -8, IL -10, IL -11, IL -12, IL -13, IL -14, IL -15, IL -16, IL -17, IL -18 usw. ein; Antigene, z.B. Peptidantigene, Proteinantigene wie z.B. Alloantigene vorzugsweise in Verbindung mit Antigenen zeigenden Zellen, Lectine, z.B. ConA, [Alpha]-CD3, LPs, usw.

Die Kultur kann auch Antikörper oder spezifische Liganden (gereinigte Liganden, FC Verschmelzungsproteinen oder andere, rekombinante Formen des Leucinzipper) für Zelloberflächenrezeptoren enthalten, die die Treg Aktivität stimulieren oder hemmen können. Im Medium können auch mAb oder Liganden, die TNFR oder andere Ko-stimulierende Moleküle auf Treg binden und die Treg Aktivität stimulieren und/oder steigern, Treg Aktivität außer Kraft setzen (und eine Vermehrung herbeiführen), oder die Apoptosis der Treg stimulieren enthalten sein. Normalerweise ist es gewollt, dass die spezifischen Kulturbedingungen einen besonderen Zweck dienen, z.B. die Erhaltung der Treg Zellaktivität usw.

Bevorzugt ist es, dass Antikörper zur Detektion des 4-1BB Rezeptors verwendet werden.

Die 4-1BB Antikörper werden einer Zellsuspension hinzugefügt und für einen ausreichenden Zeitraum inkubiert, um die verfügbaren Zelloberflächenantigene zu binden. Die Inkubation beträgt normalerweise mindestens etwa 5 Minuten bis zu 18h. Die Expression wird nach 3h bis 8h einer Stimulation gemessen. Der optimale Inkubationszeitraum ist der, innerhalb dessen möglichst viele der aktivierten, regulatorischen T-Zellen, aber möglichst wenige der nichtregulatorischen Zellen 4-1B markiert werden. Dieser Zeitrahmen kann vom Fachmann durch eine Analyse verschieden langer Inkubationszeiten während oder nach der Stimulation ermittelt werden.
Es ist wünschenswert, eine ausreichende Konzentration von Antikörpern in der Reaktionsmischung zu haben, so dass die Effizienz der Trennung nicht vom Mangel an Antikörper eingegrenzt wird. Die entsprechende Konzentration kann auch durch Titration bestimmt werden.
Das Mittel, worin die Zellen getrennt werden, kann jedes Mittel sein, das die Aktivität der Zellen erhält. Ein bevorzugtes Mittel ist eine mit Phosphat gepuffertes Salzlösung, die 0,1 bis 0,5% BSA oder in gleichen Anteilen autologes, gepooltes Serum oder Serumersatzstoffe enthält. Verschiedene Medien sind im Handel verfügbar und können abhängig vom Zellentyp und des Experimentes verwendet werden. Verfügbare Medien sind Dulbecco's modifiziertes Eagle's Medium (dMEM), Hank's Basic Salt Solution (HBSS) ein Dulbecco's Phosphat gepufferte Salzlösung (dPBS), RPMI, Iscove's Medium, PBS mit 5 mM EDTA usw. häufig ergänzte durch fötalem Kalbsserum BSA, HSA usw.

Die gekennzeichneten Zellen werden dann abhängig von der Expression von 4-1BB getrennt. Die getrennten Zellen können in jedem entsprechenden Mittel gesammelt werden, das die Lebensfähigkeit der Zellen erhält. Normalerweise werden sie in einer Sammelröhre mit etwas Serum gesammelt. Verschiedene Medien sind im Handel verfügbar und können je nach Art der identifizierten und/oder separierten Zellen verwendet werden, z.B. dMEM, HBSS einschließend, dPBS, RPMI, Iscove's Medium usw., häufig ergänzt mit fötalem Kalbsserum, autologen oder gepoolten Serum oder Serumersatzstoffen.

Des Weiteren ist bevorzugt, dass die Antigene zur Detektion des 4-1BB Rezeptors mit einem fluoreszierenden Stoff oder direkt mit magnetischen Mikropartikeln gekoppelt sind.

Für die Durchflußzytometrie oder FACS (Fluorescence activated cell sorting) und die magnetische Zellsortierung oder MACS (Magnetic activated cell sorting) werden Antikörper eingesetzt, die mit Fluoreszenzmarkern oder magnetischen Mikropartikeln gekoppelt sein können, die dem Fachmann bekannt sind, wie z.B FITC, Phycoerythrin (PE), Allophycocyanin (APC), Cascade yellow and Peridinin Chlorophyll Protein (PerCP). Die Antikörper können auch an Haptene gekoppelt sein und dann mit Hapten-spezifischen Sekundärantikörpern detektiert zu werden. Eine Kombination von Antikörpern kann benutzt werden, um verschiedene Zellen mit unterschiedlichen Eigenschaften zu detektieren, analysieren und/oder zu isolieren, auf der Basis von Markern, wie z.B. sekretorische Proteine oder charakteristische Oberflächenmoleküle. Besonders können fluoreszenzmarkierte Antikörper für FoxP3, CD25 und/oder CD4 Marker eingesetzt werden. Besonders bevorzugt können fluoreszenzmarkierte oder mit magnetischen Mikropartikeln gekoppelte 4-1BB-Antikörper eingesetzt werden. Magnetische Mikropartikel können hierbei direkt oder indirekt verwendet werden (Hapten z.B. Biotin und Anti-Hapten Mikropartikel oder Fluorochrome, z.B. PE oder APC und Anti-Fluororchrom Mikropartikel).

In einer weiteren bevorzugten Ausführungsform, ist vorgesehen, dass die regulatorischen Th-Zellen aus Blut, periphere mononukleären Blutzellen (PBMC), Körpergewebe oder Zellen der Gewebeflüssigkeit direkt identifiziert und/oder separiert bzw. isoliert werden.

Normalerweise werden die regulatorischen Th-Zellen aus Zellproben von Säugetieren (Mamalia), aber besonders von Menschen und bevorzugt aus Versuchperson und/oder Patienten identifiziert und/oder separiert. Die regulatorischen Th-Zellen können z.B. aus Blutproben, die Immunzellen enthalten (peritoneale, cerebrale-spinale, pleurale und/oder synoviale Körperflüssigkeit), Spülungsflüssigkeiten von Hohlorganen (Atemwege, Lungen), Homogenaten und Aspirata von Lymphknoten, Milz, Mandeln und/oder anderem lymphatischem Gewebe stammen. Die regulatorischen Th-Zellen können auch aus Tiergeweben gewonnen werden (z.B. von der Milz oder den Lymphknoten eines Tieres). Die Th-Zellen können auch Teil einer Blutprobe sein, z.B. von einer Versuchperson oder menschlichem Patienten, besonders bevorzugt aus peripheren mononukleären Blutzellen (PBMC), Körpergewebe oder Zellen der Gewebeflüssigkeit.

Die Erfindung sieht einen Kit vor, umfassend einen Antikörper zur Detektion des 4-1BB Rezeptors zur Identifikation und/oder Separation einer regulatorischen Th-Zelle.

Bevorzugt ist es, dass im Kit der 4-1BB Rezeptor Antikörper mit einem fluoreszierenden Stoff, mit einem Hapten oder mit magnetischen Mikropartikeln gekoppelt ist.

Für die Identifikation und/oder Separation der aktivierten, lebenden regulatorischen Th-Zellen wird ein 4-1BB Rezeptor Antikörper bereitgestellt, der an einem Fluoreszenzmarker, einem Hapten oder magnetischen Mikropartiklen gekoppelt ist. Fluoreszenzmarker sind dem Fachmann bekannt, wie z.B FITC, Phycoerythrin (PE), Allophycocyanin (APC), Cascade yellow and Peridinin Chlorophyll Protein (PerCP).

Des Weiteren ist eine bevorzugte Eigenschaft des Kits, dass aktivierte regulatorische Th-Zellen identifiziert und/oder separiert werden.

Durch die Verwendung des Kits, umfassend einen 4-1BB Rezeptor Antikörper gekoppelt mit einem Fluoreszenzmarker, Haptenen oder magnetischen Mikropartikeln können regulatorische Th-Zellen identifiziert und/oder separiert werden. Durch die Anwendung des 4-1BB Rezeptor Antikörpers können bevorzugt aktivierte regulatorische Th-Zellen identifiziert und/oder separiert werden. Besonders bevorzugt werden lebende, aktivierte regulatorische Th-Zellen identifiziert und/oder separiert.

In dieser Ausführung kann die Methode der Erfindung z.B. genutzt werden, um regulatorische Th-Zellen aus einer Blutprobe (oder aus einem Blutderivat) oder aus Zellgemischderivaten von lymphatischen Organen oder Tumoren von Säugetieren identifiziert und/oder separiert werden.

Die separierten regulatorischen Th-Zellen können vor und/oder nach dem Klonieren und/oder Vermehrung und/oder in Zellgemischen angereichert in und/oder als pharmazeutisches Mittel in der Therapie oder Vorbeugung von Krankheiten genutzt werden. Aus den separierten regulatorischen T-Zellen können ausserdem die kodierenden Gensequenzen des TCR (T-Zellrezptor) isoliert werden und für weitere therapeutische Zwecke wie zB für Krebstherapien genutzt werden. Außerdem können die in der genannten Form vorliegenden regulatorischen Th-Zellen in weiteren Untersuchungen und/oder Analysen eingesetzt werden. Das pharmazeutische Mittel kann für die Behandlung und/oder Vorbeugung von Krankheiten in Säugetieren verwendet werden, was eine Verabreichung einer pharmazeutisch wirksamen Menge des pharmazeutischen Mittels in das Säugetier einschließen kann.

Die Krankheit kann irgendeine Krankheit sein, die durch die Gegenwart von einer separierten Zelle und/oder durch die Konzentrationserhöhung der betreffenden Zellen in/an der betreffenden Stelle, oder im gesamten Säugetierprobanden und/oder Patienten behandelt und/oder vorgebeugt werden kann. Zum Beispiel kann die Zelle eine regulatorische Th-Zelle sein und die behandelte und/oder präventiv behandelte Krankheit kann eine Autoimmunkrankheit, eine infektiöse Krankheit, eine Allergie, Transplantat versus Wirtskrankheit (oder allogene Transplantatabstoßung) und/oder irgendeine andere durch Hypersensitivität eingeleitete Krankheit sein.

Krankheiten, wofür die in der Erfindung dargelegte Verwendung besonders geeignet ist, sind solche, die durch und/oder während einer fehlenden Regulation der Immunantwort entstehen. Diese Krankheiten können Transplantatabstoßungen sein, allergische Zustände, bestimmte infektiöse Krankheiten und/oder Autoimmunkrankheiten sein.

Im Zusammenhang mit Autoimmunkrankheiten ist es bekannt, dass das Immunsystem körpereigene Strukturen attackiert, wie z.B. bei rheumatischer Arthritis, Insulin abhängiger Diabetes mellitus (IDDM), Multiple Sklerose (MS), Atherosklerose, Psoriasis, allogener Stammzellentransplantation, Organtransplantation. Eine Therapie mit spezifischen regulatorischen T-Zellen ist hier vorteilhaft. Bei Tumorerkrankungen sollen regulatorische T-Zellen spezifisch für Tumorantigene eliminiert werden, so dass das. Immunsystem effiziente anti-Tumor Immunantworten initieren kann.

Die Erfindung betrifft auch die Verwendung der erfindungsgemäßen Zusammensetzung und/oder des erfindungsgemäßen pharmazeutischen Mittels zur Behandlung von Krankheiten, die mit einer Defizienz der zellulären Immunität verbunden sind, zum Beispiel bei dem Defekt nach ICDIO-Code: D.84.4.
Hierbei kann es sich um Sepsiserkrankungen handeln, um Entzündungs-reaktionen und Fieber, um Autoimmun-Erkrankungen und Erkrankungen der Störung der Zellteilung wie zum Beispiel Krebs.
Entzündungen im Sinne der Erfindung sind die vom Bindegewebe und den Blutgefäßen getragene Reaktion des Organismus auf einen äußeren oder innerlich ausgelösten Entzündungsreiz mit dem Zweck, diesen zu beseitigen oder zu inaktivieren und die reizbedingte Gewebsschädigung zu reparieren. Auslösend wirken mechanische Reize (Fremdkörper, Druck, Verletzung) und andere physikalische Faktoren (ionisierende Strahlen, UV-Licht, Wärme, Kälte), chemische Stoffe (Laugen, Säuren, Schwermetalle, bakterielle Toxine, Allergene und Immunkomplexe) sowie Erreger(Mikroorganismen, Würmer, Insekten) bzw. krankhafte Stoffwechselprodukte, entgleiste Enzyme, bösartige Tumoren. Das Geschehen beginnt mit einer kurzen Arteriolenverengung (durch Adrenalinwirkung) mit Mangeldurchblutung und Gewebsalteration, gefolgt von der Entwicklung der klassischen örtlichen Entzündungszeichen (Kardinalsymptome; nach GALEN und CELSUS), das heißt von Rötung (= Rubor; Gefäßerweiterung durch Histamin), Wärme (= Calor; durch örtliche Stoffwechselsteigerung), Schwellung (= Tumor; durch Austritt eiweißreicher Flüssigkeit aus den - unter anderem durch Histamin - veränderten Gefäßwänden, unterstützt durch die verlangsamte Blutzirkulation im Sinne der Prästase bis Stase), Schmerz (= Dolor; als Folge der erhöhten GewebsSpannung und schmerzauslösender Entzündungsprodukte, zum Beispiel Bradykinin) und Funktionsstörung (= Functio laesa). Der Vorgang wird ergänzt durch Störung des Elektrolythaushaltes (Transmineralisation), Einwanderung neutrophiler Granulozyten und Monozyten durch die Gefäßwände (s.a. Leukotaxis), letzteres mit dem Zweck, den Entzündungsreiz und geschädigte bis neukrotische Zellen zu beseitigen (Phagozytose); ferner wandern Lymphozyten-Effektorzellen ein, die zur Bildung spezifischer Antikörper gegen den Entzündungsreiz führen (Immunreaktion), sowie Eosinophile (in der Heilungsphase bzw. - sehr frühzeitig - bei allergisch-hyperergischem Geschehen). Durch die bei der Reaktion erfolgende Aktivierung des Komplementsystems werden Bruchstücke (C3a und C5a) dieses Systems frei, die - wie das Histamin und Bradykinin - als Mediatoren der Entzündung wirken, und zwar im Sinne der Anregung der Chemotaxis der zitierten Blutzellen; ferner wird die Blutgerinnung aktiviert. In der Folge tritt eine Schädigung (Dystrophie und Koagulations-nekrose) des zugeordneten Organparenchyms ein. Der Gesamt-Organismus reagiert je nach Intensität und Art der Entzündung mit Fieber, Stress (s.a. Adaptationssyndrom), Leuko-zytose und Veränderungen in der Zusammensetzung der Plasmaproteine (Akute-Phase-Reaktion), die zu einer beschleunigten Blutkörperchensenkungsreaktion führen. Bevorzugte Entzündungen im Sinne der Erfindung sind die eitrige, die exudative, die fibrinöse, die gangräneszierende, die granulomatöse, die hämorrhagische, die katarrhalische, die nekrotisierende, die proliferative oder produktive, die pseudomembranöse, die seröse, die spezifische und/oder die ulzeröse Entzündungen.

Autoimmunerkrankungen im Sinne der Erfindung sind Krankheiten, die zum Teil auf die Bildung von Autoantikörpern und deren schädigende Einwirkung auf den Gesamtorganismus bzw Organsysteme, das heißt auf Autoaggression zurückzuführen sind. Andererseits kann es sich bei Autoimmunerkrankungen auch um Krankheiten halten, bei denen T-Zellen eine herausragende Rolle bei der Pathogenese bzw. Initation innehaben. Eine Klassifikation ist als organspezifische, intermediäre und/oder systemische Autoimmunerkrankung möglich. Bevorzugte organspezifische Autoimmunerkrankungen sind HASHIMOTO Thyreoiditis, primäres Myxödem, Thyreotoxikose (BA-SEDOW Krankheit), perniziöse Anämie, ADDISON Krankheit, Myasthenia gravis und/oder juve-niler Diabetes mellitus. Bevorzugte intermediäre Autoimmunkrankheiten sind GOODPASTURE Syndrom, autoimmune hämolyti-sche Anämie, autoimmune Leukopenie, idiopathische Thrombo-zytopenie, Pemphigus vulgaris, sympathische Ophthalmie, primäre biliäre Zirrhose, Autoimmunhepatitis, Colitis ulcerosa und/oder SJÖGREN Syndrom. Bevorzugte systemische Autoimmunkrankheiten sind rheumatoide Arthritis, rheumatisches Fieber, systemischer Lupus e-rythematodes, Dermato-myositis/Polymyositis, progressive systemische Sklerose, WEGENER Granulomatose, Panarteriitis nodosa und/oder Hyper-sensitivitätsangiitis. Typische Autoimmunkrankheiten sind Thyreotoxikose, Schilddrüsenbedingtes Myxödem, HASHIMOTO Thyreoiditis, generalisierte Endokrinopathie, perniziöse Anämie, chronische Gastritis Typ A, Krankheiten einzelner oder aller korpuskularen Elemente des Blutes (zum Beiapiel autoimmunhämolytische Anämie, idiopath. Thrombozytopenie bzw. -pathie; idiopath. Leukopenie bzw. Agranulozytose), Pemphigus vulgaris und Pemphigoid, sympathische Ophthalmie und manche Uveitis-Formen, primär biliäre Leberzirrhose und chronisch aggressive Autoimmunhepatitis, Diabetes mellitus Typ I, CROHN Krankheit und Colitis ulcerosa, SJÖGREN Syndrom, ADDISON Krankheit, Lupus e-rythematodes disseminatus und als diskoide Form dieser Krankheit, als Dermatomyositis und Sklerodermie, rheumatoide Arthritis (= primär-chronische Polyarthritis), Antiglomerulusbasalmembran-Nephritis. Grundlage sind eine aggressive Immunreaktion infolge Zusammenbruchs der Immuntoleranz gegenüber Selbst-Derterminanten und ein Übergewicht von inflammatorischen T-Helfer-zellen. Ferner ist die Bildung von Autoantigenen möglich, zum Beispiel durch Verbindung von Wirtsproteinen mit Haptenen (zum Beispiel Arzneimittel), durch ontogenetisches Gewebe, das sich erst nach Entwicklung der Selbsttoleranz entwickelt und für durch Änderungen der Konformation der Proteine demaskierte Proteinkomponenten im Zusammenhang zum Beispiel mit Infektion durch Viren oder Bakterien; ferner für im Zusammenhang mit Neoplasien entstandene neue Proteine.

Sepsiserkrankungen sind im Sinne der Erfindung Erkrankungen infolge dauernden oder periodischen Eindringens von pathogenen Bakterien und/oder deren Giften aus einem Krankheitsherd und deren Ausbreitung auf dem Lymph-Blut-Weg zur allgemein beziehungsweise lokalen Infektion.
Sepsis im Sinne der Erfindung sind bevorzugt Wundsepsis (Phlegmone, Thrombophlebitis, Lymphangitis), Puerperalsepsis (bei Puerperalfieber), otogene Sepsis (bei Otitis media), tonsillogene Sepsis (bei Angina, Peritonsillitis), cholan-gitische Sepsis (bei eitriger Cholezystzitis, Cholangitis), pylephlebitische Sepsis (bei Pylephlebitis) Nabelsepsis (bei Omphalitis etc.), Urosepsis sowie bei Zahngranulom. Die Sepsis im Sinne der Erfindung kann akut bis hochakut (foudroyant), subakut (z. Beispiel als Endocarditits lenta) oder chronisch erfolgen, aber selbstverständlich auch als Neugeborenensepsis.
Sepsis im Sinne der Erfindung sind also alle pathogenen Veränderungen eines Patienten, die mit intermitterierendem Fieber und Schüttelfrost verbunden sein können sowie mit Milztumor, mit toxischen Reaktionen beziehungsweise Schäden des Knochenmarks beziehungsweise des Blutes (polynukleäre Leukozytose, Anämie, Hämolyse, Thrombozytopenie) oder aber mit pathogenen Reaktionen am Herzen und der Gefäßnerven (Tachykardie, Zentralisation des Kreislaufs, Ödeme, Oligurie; evtl. Schock) beziehungsweise des Verdauungstraktes (trockene, belegte Zunge, Durchfälle) oder aber mit Septikopyämie (Pyämie mit Bildung septischer Infarkte und metastatischer Abszess) .

Bevorzugte Erkrankungen, die mit einer Defizien des zellulären Immunsystems verbunden sind, sind im Sinne der Erfindung weiterhin:
AIDS, Akne, Albuminurie (Proteinurie), Alkoholentzugssyndrom, Allergien, Alopezie (Haarausfall), ALS (Amyotrophe Lateralsklerose), Alzheimer Erkrankung, AMD (Altersbedingte Makuladegeneration), Anämie, Angststörungen, Anthrax (Milzbrand), Aortensklerose, arterielle Verschlusskrankheit, Arterienverkalkung, Arterienverschluss, Arteriitis temporalis, Arteriosklerose, Arteriovenöse Fisteln, Arthritis, Arthrose, Asthma, Ateminsuffizienz, Autoimmunerkrankung, AV-Block, Azidose, Bandscheibenvorfall, Bauchfellentzündung, Bauchspeicheldrüsenkrebs, Becker Muskeldystrophie, Benigne Prostata Hyperplasie (BPH), Blasenkarzinom, Bluterkrankheit (Hämophilie), Bronchialkarzinom, Brustkrebs, BSE, Budd-Chiari-Syndrom, Bulimia nervosa, Bursitis (Schleimbeutelentzündung), Byler-Syndrom, Bypass, Chlamydien-Infektion, Chronische Schmerzen, Cirrhose, Commotio cerebri (Gehirnerschütterung), Creutzfeld-Jakob-Erkrankung, Darmkarzinom, Darmkrebs, Darmtuberkulose, Depression, Diabetes insipidus, Diabetes mellitus, Diabetes mellitus juvenilis, Diabetische Retinopathie, Duchenne-Muskeldystrophie, Duodenalkarzinom, Dystrophia musculorum progressiva, Dystrophie, Ebola, Ekzem, Erektile Dysfunktion, Fettsucht, Fibröse, Gebärmutterhalskrebs, Gebärmutterkrebs, Gehirnblutung, Gehirnentzündung, Haarausfall, Halbseitenlähmung, Hämolytische Anämie, Hämophilie, Haustierallergie (Tierhaarallergie), Hautkrebs, Herpes zoster, Herzinfarkt, Herzinsuffizienz, Herzklappenentzündung, Hirnmetastase, Hirnschlag, Hirntumor, Hodenkrebs, Ischämie, Kahler-Krankheit (Plasmozytom), Kinderlähmung (Poliomyelitis), Knochenschwund, Kontaktekzem, Lähmung, Leberzirrhose, Leukämie, Lungenfibrose, Lungenkrebs, Lungenödem, Lymphdrüsenkrebs (Morbus Hodgkin), Lymphogranulomatose, Lymphom, Lyssa, Magenkarzinom, Mammakarzinom, Meningitis, Milzbrand, Mukoviszidose (zystrische Fibröse), Multiple Sklerose (MS), Myokardinfarkt, Neurodermitis, Neurofibromatose, Neuronale Tumoren, Nierenkrebs (Nierenzellkarzinom), Osteoporose, Pankreaskarzinom, Pneumonie, Polyneuropathien, Potenz-Störungen, Progressive Systemische Sklerose (PSS), Prostatakrebs, Quaddelauschlag (Urtikaria), Querschnittssyndrom, traumatisches, Rektumkarzinom, Rippenfellentzündung, Schädel-Hirn-Trauma, Scheidenkrebs (Vaginalkarzinom), Sinusitis, Speiseröhrenkrebs, Tremor, Tuberkulose, Tumorschmerz, Vaginalkarzinom, Verbrennung - Verbrühung, Vergiftungen, Virale Meningitis, Wechseljahre, Weichteilsarkom, Weichteiltumor, Zerebrale Durchblutungsstörungen und/oder ZNS-Tumore.

In einer bevorzugten Ausführungsform ist die Krebserkrankung oder der Tumor, die/der behandelt oder verhindert wird, ausgewählt aus der Gruppe von Krebserkrankungen oder Tumorerkrankungen des Hals-Nasen-Ohren-Bereichs, der Lunge, des Mediastinums, des Gastrointestinaltraktes, des Urogenital-Systems, des gynäkologischen Systems, der Brust, des endokrinen Systems, der Haut, Knochen- und Weichteilsarkomen, Mesotheliomen, Melanomen, Neoplasmen des zentralen Nervensystems, Krebserkrankungen oder Tumorerkrankungen im Kindes-alter, Lymphomen, Leukämien, paraneoplastischen Syndromen, Metastasen ohne bekannten Primärtumor (CUP-Syndrom), perito-nealen Karzinomastosen, Immunsuppression-bezogenen Maligni-täten und/oder Tumor-Metastasen.
Insbesondere kann es sich bei den Tumoren um folgende Krebsarten handeln: Adenokarzinom der Brust, der Prostata und des Dickdarms; alle Formen von Lungenkrebs, der von den Bronchien ausgeht; Knochenmarkkrebs, das Melanom, das Hepatom, das Neuroblastom; das Papillom; das Apudom, das Choristom, das Branchiom; das maligne Karzinoid-Syndrom; die Karzinoid-Herzerkrankung; das Karzinom (zum Beispiel Walker-Karzinom, Basalzellen-Karzinom, basosquamöses Karzinom, Brown-Pearce-Karzinom, duktales Karzinom, Ehrlich-Tumor, in situ-Karzi-nom, Krebs-2-Karzinom, Merkel-Zellen-Karzinom, Schleimkrebs, nicht-kleinzelliges Bronchialkarzinom, Haferzellen-Karzinom, papilläres Karzinom, szirrhöses Karzinom, bronchiolo-alveo-läres Karzinom, Bronchiai-Karzinom, Plattenepithelkarzinom und Transitionalzell-Karzinom); histiocytische Funktionsstörung; Leukämie (zum Beispiel in Zusammenhang mit B-Zellen-Leukämie, Gemischt-Zellen-Leukämie, Nullzeilen-Leukämie, T-Zellen-Leukämie, chronische T-Zellen-Leukämie, HTLV-II-assoziierte Leukämie, akut lymphozytische Leukämie, chronisch- lymphozythische Leukämie, Mastzell-Leukämie und myeloische Leukämie); maligne Histiocytose, Hodgkin-Krankheit, non-Hodgkin-Lymphom, solitärer Plasmazelltumor; Reti-culoendotheliose, Chondroblastom; Chondrom, Chondrosarkom; Fibrom; Fibrosarkom; Riesenzell-Tumore; Histiocytom; Lipom; Liposarkom; Leukosarkom; Mesotheliom; Myxom; Myxosarkom; Osteom; Osteosarkom; Ewing-Sarkom; Synoviom; Adenofribrom; Adenolymphom; Karzinosarkom, Chordom, Craniopharyngiom, Dysgerminom, Hamartom; Mesenchymom; Mesonephrom, Myosarkom, Ameloblastom, Cementom; Odontom; Teratom; Thymom, Chorio-blastom; Adenokarzinom, Adenom; Cholangiom; Cholesteatom; Cylindrom; Cystadenocarcinom, Cystadenom; Granulosa-Zelltumor; Gynadroblastom; Hidradenom; Inselzelltumor; Leydig-Zelltumor; Papillom; Sertoli-Zeil-Tumor, Thekazelltumor, Leiomyom; Leiomyosarkom; Myoblastom; Myom; Myosarkom; Rhabdomyom; Rhabdomyosarkom; Ependynom; Ganglioneurom, Gliom; Medulloblastom, Meningiom; Neurilemmom; Neuroblastom; Neuroepitheliom, Neurofibrom, Neurom, Paragangliom, nicht-chromaffines Paragangliom, Angiokeratom, angiolymphoide Hyperplasie mit Eosinophilie; sclerosierendes Angiom; Angiomatose; Glomangiom; Hemangioendotheliom; Hemangiom; Hemangiopericytom, Hemangiosarkom; Lymphangiom, Lymphangiomyom, Lymphangiosarkom; Pinealom; Cystosarkom phyllodes; Hemangiosarkom; Lymphangiosarkom; Myxosarkom, Ovarialkarzinom; Sarkom (zum Beispiel Ewing-Sarkom, experimentell, Kaposi-Sarkom und Mastzell-Sarkom); Neoplasmen (zum Beispiel Knochen-Neoplasmen, Brust-Neoplasmen, Neoplasmen des Verdauungssystems, colorektale Neoplasmen, Leber-Neoplasmen, Pankreas-Neoplasmen, Hirnanhang-Neoplasmen, Hoden-Neoplasmen, Orbita-Neoplasmen, Neoplasmen des Kopfes und Halses, des Zentralnervensystems, Neoplasmen des Hörorgans, des Beckens, des Atmungstrakts und des Urogenitaltrakts); Neurofibromatose und zervikale Plattenepitheldysplasie.

In einer weiter bevorzugten Ausführungsform ist die Krebserkränkung oder der Tumor, die/der behandelt oder verhindert wird, ausgewählt aus der Gruppe: Tumoren des Hals-Nasen-Ohren-Bereichs umfassend Tumoren der inneren Nase, der Nasennebenhöhlen, des Nasopharynx, der Lippen, der Mundhöhle, des Oropharynx, des Larynx, des Hypopharynx, des Ohres, der Speicheldrüsen und Paragangliome, Tumoren der Lunge umfassend nicht-kleinzellige Bronchialkarzinome, kleinzellige Bronchialkarzinome, Tumoren des Mediastinums, Tumoren des Gastrointestinaltraktes umfassend Tumoren des Ösophagus, des Magens, des Pankreas, der Leber, der Gallenblase und der Gallenwege, des Dünndarms, Kolon- und Rektumkarzinome und Analkarzinome, Urogenitaltumoren umfassend Tumoren der Nieren, der Harnleiter, der Blase, der Prostata, der Harnröhre, des Penis und der Hoden, gynäkologische Tumoren umfassend Tumoren des Zervix, der Vagina, der Vulva, Korpuskarzinom, maligne Trophoblastenerkrankung, Ovarialkar-zinom, Tumoren des Eileiters (Tuba Faloppii), Tumoren der Bauchhöhle, Mammakarzinome, Tumoren endokriner Organe umfassend Tumoren der Schilddrüse, der Nebenschilddrüse, der Nebennierenrinde, endokrine Pankreastumoren, Karzinoidtumoren und Karzinoidsyndrom, multiple endokrine Neoplasien, Knochen- und Weichteilsarkome, Mesotheliome, Hauttumoren, Melanome umfassend kutane und intraokulare Melanome, Tumoren des zentralen Nervensystems, Tumoren im Kindesalter umfassend Retinoblastom, Wilms Tumor, Neurofibromatose, Neuroblastom, Ewing-Sarkom Tumorfamilie, Rhabdomyosarkom, Lymphome umfassend Non-Hodgkin-Lymphome, kutane T-Zell-Lymphome, primäre Lymphome des zentralen Nervensystems, Morbus Hodgkin, Leukämien umfassend akute Leukämien, chronische myeloische und lymphatische Leukämien, Plasmazell-Neoplasmen, myelodysplastische Syndrome, paraneoplastische Syndrome, Metastasen ohne bekannten Primärtumor (CUP-Syndrom), peritoneale Karzinomastose, Immunsuppressionbezogene Malignität umfassend AIDS-bezogene Malignitäten wie Kaposi-Sarkom, AIDS-assoziierte Lymphome, AIDS-assoziierte Lymphome des zentralen Nervensystems, AIDS-assoziierter Morbus Hodgkin und AIDS-assoziierter anogenitale Tumoren, Trans-plantations-bezogene Malignitäten, metastasierte Tumoren umfassend Gehirnmetastasen, Lungenmetastasen, Lebermetastasen, Knochenmetastasen, pleurale und perikardiale Metastasen und maligne Aszites.
In einer weiter bevorzugten Ausführungsform ist die Krebserkrankung oder der Tumor, die/der behandelt oder verhindert wird, ausgewählt aus der Gruppe umfassend Krebserkrankungen oder Tumorerkrankungen der Mammakarzinome, der Gastrointes-tinaltumore, einschließlich Kolonkarzinome, Magenkarzinome, Pankreaskarzinome, Dickdarmkrebs, Dünndarmkrebs, der Ovarialkarzinome, der Zervikalkarzinome, Lungenkrebs, Prostatakrebs, Nierenzellkarzinome und/oder Lebermetastasen.

Immunopathologische Reaktionen verursachen den Funktionsverlust eines bestimmten Gewebes und können Gewebe zerstören. Die erfindungsgemäße Verwendung erlaubt es aktive regulatorische Th- Zellen von Patienten mit diesen Krankheiten zu isolieren, um die gewebeschädigende Immunantwort zu zu behandeln. Bei Tumorerkrankungen soll die erfindungsgemäße Verwendung eine spezifische Elimierung von Tumorspezifischen Treg in vitro oder ex vivo ermöglichen. Falls die regulatorischen Th-Zellen in einer zu geringen Anzahl vorliegen, können sie separiert und angereichert werden und/oder in Zellkulturen vermehrt werden. In beiden Fällen könnte der Patient mit der angereicherten und/oder vermehrten und somit erhöhten Anzahl von regulatorischen Th-Zellen behandelt werden, wodurch eine Unterdrückung der immunopathologischen Antwort und somit eine Verbesserung und/oder Heilung der Krankheit erwartet würde. Autoimmunkrankheiten, wofür der 4-1BB Rezeptors als Marker zur Identifikation und/oder Isolierung und folgender Anreicherung von aktiven regulatorischen Th-Zellen verwendet werden könnte und ein erfolgreiche Therapie erwartet würde, beinhalten, aber nicht nur auf diese beschränkt, Diabetes mellitus (IDDM), Multiple Sklerose (MS), Atherosklerose (AS), Psoriasis, Darmentzündungen, autoimmunbedingte haemolytische Anämie, Sjogrenssyndrom, autoimmunbedingte Thyroiditis und systemtische Lupus Erythematosis.

Dem Fachmann ist bekannt, dass regulatorische Th-Zellen im Zusammenhang mit Transplantationen fähig sind eine Immunantworten zu unterdrücken, die zu einer Transplantatabstoßung führen könnte. Die Behandlung eines Patienten und/oder Probanden mit zunächst isolierten und dann angereicherten spezifisch aktivierten regulatorischen Th-Zellen könnte eine Transplantatabstoßung vorbeugen und/oder verhindern. Transplantatabstoßungen werden durch eine Immunantwort des Transplantatakzeptors ausgelöst. Hierbei bekämpfen die Immunzellen des Akzeptors das körperfremde Gewebe. In dieser Anwendungsform würden Th-Zellen des Zielpatienten in vitro Zellen, Zellextrakten, isolierten Antigenen oder Proteinen des Organspenders vor oder nach der Transplantation ausgesetzt werden. Hiernach würden die aktiven regulatorischen Th-zellen nach einer angebrachten Vermehrungszeit anhand des 4-1BB Markers identifiziert und/oder separiert werden. Die identifizierten und/oder separierten aktiven regulatorischen Th-Zellen würden dann dem Transplantatakzeptor wieder zugeführt werden. Es wird so erreicht, dass die nach dieser Methode behandelten Zellen die Immunantwort gegen das Transplantat unterbinden und die Transplantatabstoßung verzögert oder verhindert wird. Die Verwendung des 4-1BB Markers könnte unter anderen bei Allogentransplantaten und/oder Heterotransplantaten, wie z.B. bei Herz-, Lungen-, Darm-, Kornea-, Nieren- und Knochenmarktransplantationen eingesetzt werden.

Im Falle einer Allergie, würden regulatorische Th-Zellen entweder vor der polyklonalen Aktivierung der T-Zellen oder nach der Kultivierung mit spezifischen Allergenen identifiziert und/oder separiert werden. Die identifizierten und/oder separierten Zellen würden dann nach einer eventuellen Vermehrung dem Patienten zurückgegeben. Es wird nun erwartet, dass die so behandelten regulatorischen Th-Zellen die Immunantwort unterdrücken und sich die zu den verwendeten Antigenen korrelierende Symptomatik verbessert.

Die Behandlung von Allergien anhand der Identifizierung und/oder Isolierung von aktiven regulatorische Th-Zellen mit dem Marker 4-1BB und der beschriebenen Methode würde sich unter anderen bei Hautauschlägen, atopischer Dermatitis, Asthma, allergischer Rhinitis, Insektengiften und Lebensmittelallergie wie z.B. gegen Gluten, Milchprodukten, Nüssen und/oder Fischantigenen eignen.

Bei infektiösen Erkrankungen ist es von Bedeutung die Immunantwort zu stärken als sie zu schwächen. In bestimmten Krankheiten ist es von belang die Immunantwort zu modulieren um den Krankheitsverlauf zu verändern. Solche Fälle der Modulation treten bei Krankheiten auf, wo durch die Infektion immunopathologische Reaktionen auftreten, die Gewebe zerstören und bei denen wo die Immunantwort den Körper nicht schützt. Durch die Identifizierung und/oder Isolierung von aktiven regulatorischen Th-Zellen könnte die Immunantwort unterdrückt werden und immunopathologische Schäden gelindert und/oder verhindert werden.

Eine weitere Alternative, wäre die Behandlung mit aus einem Patienten isolierten regulatorischen Th-Zellen, die bestimmten Antigenen ausgesetzt wurden, um regulatorische Th-Zellen zu erhalten, die die Immunantwort in eine definierte, bestimmte Richtung umschalten könnten.

Gene können in die Zellen vor Kultur oder Transplantation für eine Vielfalt von Zwecken eingeführt werden, z.B. um die Empfänglichkeit einer Infektion zu verhindern oder zu reduzieren, Gene zu ersetzen, die einen Verlust einer Funktionsmutation unterliegen, die Fähigkeit der Treg zu steigern, um Th-Zellen zu hemmen usw. Des Weiteren können Vektoren eingeführt werden die, antisense mRNA oder Ribozyme eprimieren, wodurch die Expression eines unerwünschten Gens blockiert wird. Andere Methoden einer Gentherapie sind die Einführung von Medikamentenwiderstandsgenen, zum Beispiel das Medikamentenwiderstandsgen (MDR) oder Antiapoptosis Genen, wie bcl -2. Weiterhin können Techniken angewendet werden, die dem Fachmann bekannt sind, um Zielzellen zu transfizieren, z.B. durch Elektroporation, Kalzium gefällte DNA, Verschmelzung, Transfektion oder Lipofektion. Die besondere Art, in der die DNA eingeführt wird, ist für die Verwendung der Erfindung nicht entscheidend.

Dem Fachmann sind viele Vektoren bekannt, die verwendet werden können, um exogene Gene in Säugetierzellen zu übertragen. Die Vektoren können episomal sein, z.B. Plasmide, Virus Vektoren wie z.B. Zytomegalovirus, Adenovirus usw. Außerdem können die Gebe durch homologe Rekombination oder durch zufällige Integration ins Zielzellgenom integriert werden, wie z.B. von Retrovieren abgeleitete Vektoren wie MMLV, HIV -1, ALV, usw.

Anhand von in vitro Assays oder Screenings können aktive Faktoren der Treg-Zellen analysiert werden. Es können auch Co-culture Assays durchgeführt werden, um die Veränderungen von Tregs zu studieren, die die Vermehrung von normalen T Zellen einschließlich CD4 T als auch CD8 T zu suprimieren.
Interaktionen mit dendritischen Zellen und anderen Antigen determinierten Zellen können ebenfalls untersucht werden.
Die nach der Erfindung separierten regulatorischen Zellen können das Ausgangsmaterial einer großen Vielfalt von unterschiedlichen Analysen sein, z.B. von immunoassays für Proteinbindungsstudien, Bestimmungen des Zellwachstums, Differenzierung und funktionelle Aktivität, Produktion von Hormonen usw.

### Die Figuren stellen folgendes dar:

Figuren 1a und 1b
   *4-1BB wird wird früh nach Aktivierung spezifisch von regulatorischen T-Zellen ausgeprägt*
   Verschiedene Subpopulationen (I-IV) peripherer Th-Zellen (A) wurden mittels FACS isoliert aus PBMC (B) und an Aliquots intrazelluläre Analysen des für regulatorische T-Zellen spezifischen Transkriptionsfaktors Foxp3 durchgeführt (C).
   Die isolierten Subpopulationen wurden dann mit PMA / Ionomycin in vitro für unterschiedlich lange Zeitspannen (0, 4 und 8 Stunden) stimuliert und die Expression von 4-1BB mittels FACS analysiert (D).
Figur 2
   *Der für regulatorische T-Zellen spezifische Transkriptionsfaktor Foxp3 wird nach Aktivierung spezifisch in 4-1BB⁺ Th-Zellen ausgeprägt (RNA-Analyse)*
   PBMC wurden für 4h mit dem Superantigen SEB in vitro stimuliert und nachfolgend mit FACS 4-1BB⁺ und 4-1BB⁻ Th-Zellen isoliert. Aus den isolierten Zellen wurde RNA isoliert und mittels RT-PCR die Expression von Foxp3 analysiert.
Figur 3
   *Der für regulatorische T-Zellen spezifische Transkriptionsfaktor Foxp3 wird nach Aktivierung spezifisch in 4-1BB⁺ Th-Zellen ausgeprägt (Protein-Analyse)*
   CD4⁺ Th-Zellen wurden aus PBMC isoliert und für 4h mit dem PMA / Ionomycin in vitro stimuliert. Nachfolgend wurden mit FACS 4-1BB⁺ und 4-1BB⁻ Th-Zellen isoliert. Die isolierten Zellen wurden dann intrazellulär mit Foxp3-spezifischen Antikörpern markiert.
Figur 4
   *Koexpression von 4-1BB und Foxp3 früh nach Aktivierung*
   CD4⁺ Th-Zellen wurden aus PBMC isoliert und für 4h mit dem PMA / Ionomycin in vitro stimuliert. Nachfolgend wurden die Zellen extrazellulär mit Antikörpern gegen CD69 (Aktivierungsmarker) und 4-1BB markiert. Nachfolgend wurde intrazellulär mit Foxp3-spezifischen Antikörpern markiert. Im linken Analyseplot ist das elektronische Analysefenster für CD69⁺ aktivierte Th-Zellen angezeigt. Im rechten Analyseplot ist die Koexpression von 4-1BB und Foxp3 in CD69⁺ aktvierten Th-Zellen dargestellt.

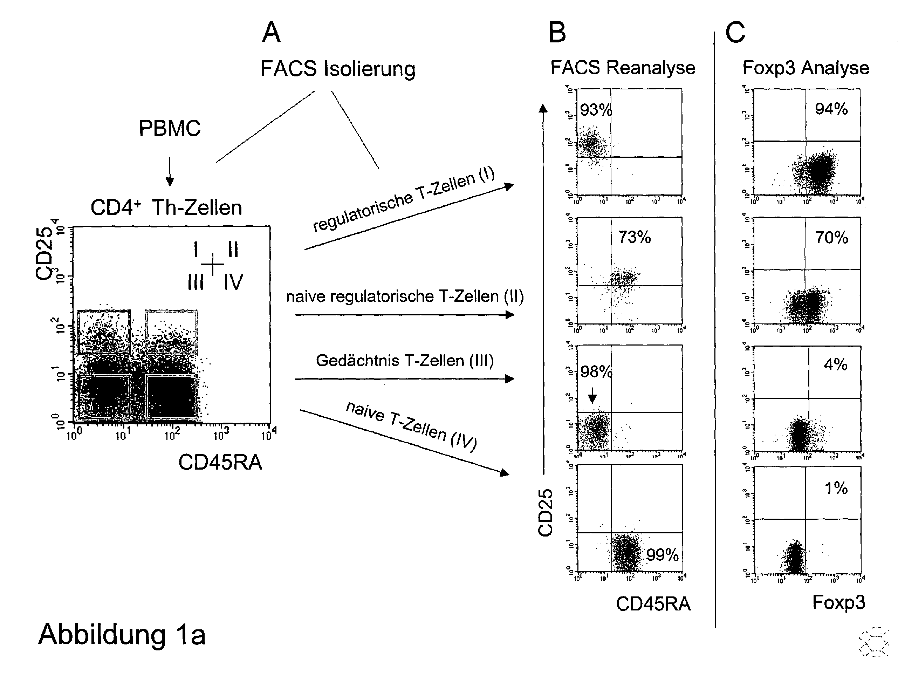

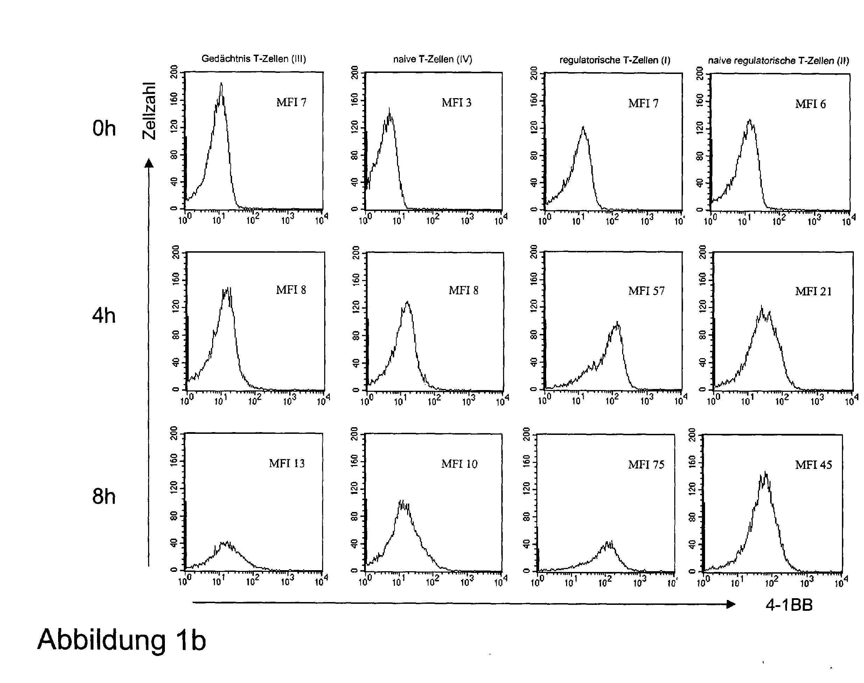

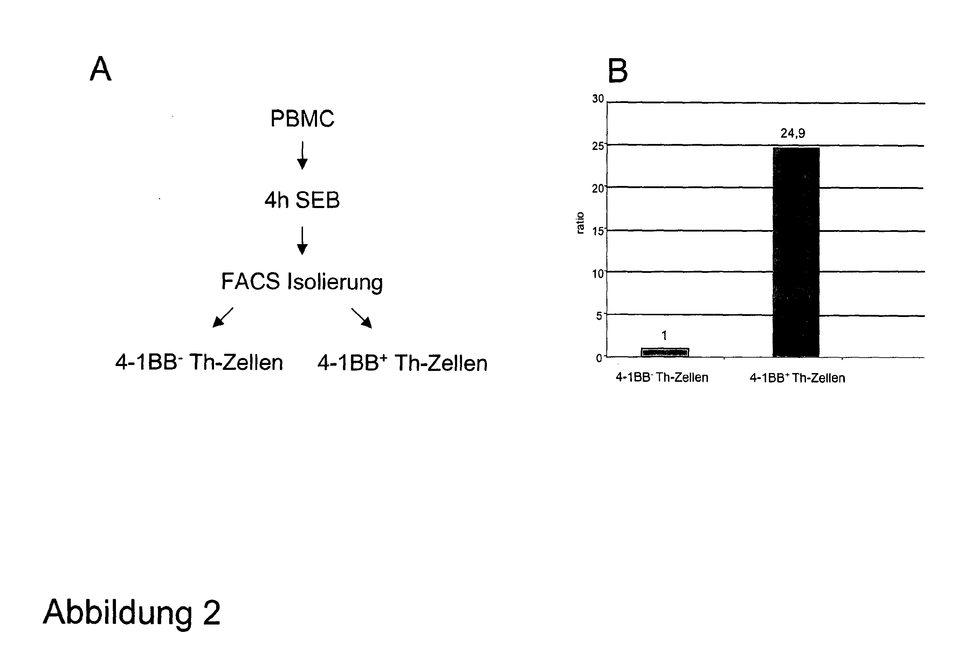

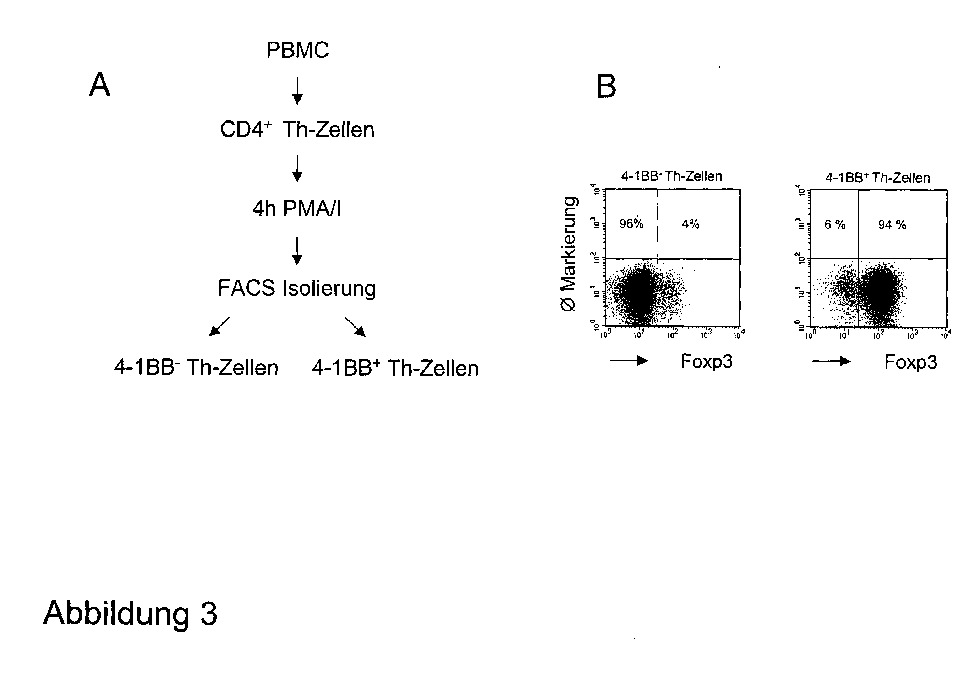

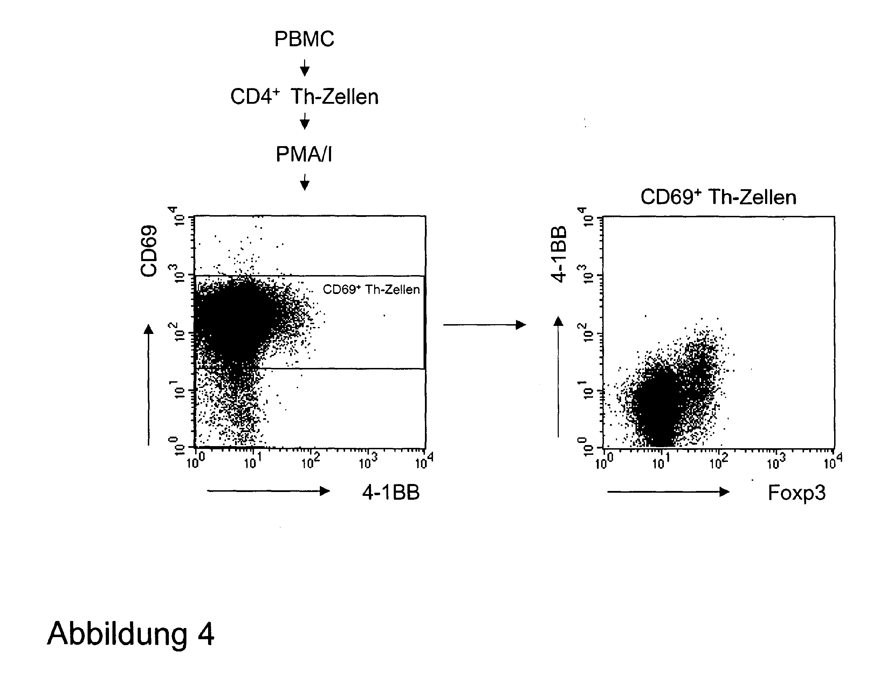

## Patentansprüche

1. Verwendung von einem 4-1BB Rezeptor zur Identifizierung und/oder Separation regulatorischer Th-Zellen (Treg).

2. Verwendung nach Anspruch 1
**gekennzeichnet dadurch, dass**
die regulatorischen Th-Zellen aus einem Zellgemisch identifiziert und/oder separiert werden.

3. Verwendung nach Anspruch 1 oder 2,
**dadurch gekennzeichnet, dass**
aktivierte regulatorische Th-Zellen identifiziert und/oder separiert werden.

4. Verwendung nach einem der Ansprüche 1 bis 3,
**dadurch gekennzeichnet, dass**
CD4+, CD25+ und/oder FoxP3+ regulatorische Th-Zellen identifiziert und/oder separiert werden.

5. Verwendung nach einem der vorherigen Ansprüche
**dadurch gekennzeichnet, dass**
lebende regulatorische Th-Zellen identifiziert und/oder separiert werden.

6. Verwendung nach einem der vorherigen Ansprüche,
**dadurch gekennzeichnet, dass**
Antigen-spezifische regulatorische Th-Zellen identifiziert und/oder separiert werden.

7. Verwendung nach einem der vorherigen Ansprüchen,
**dadurch gekennzeichnet, dass**
die regulatorischen Th-Zellen durch Durchflußzytometrie oder magnetische Zellsortierung identifiziert und/oder separiert werden.

8. Verwendung nach einem der vorherigen Ansprüchen,
**dadurch gekennzeichnet, dass**
die Expression des 4-1BB Rezeptors nach und/oder während einer Stimulation gemessen wird.

9. Verwendung nach einem der vorherigen Ansprüchen,
**dadurch gekennzeichnet, dass**
die regulatorischen Th-Zellen nach und/oder während einer Stimulation der Zellen im Zellgemisch identifiziert und/oder separiert werden.

10. Verwendung nach einem der vorherigen Ansprüchen
**dadurch gekennzeichnet, dass**
die Zellen durch Wachstumsfaktoren, Antikörpern und/oder Liganden stimuliert werden.

11. Verwendung nach einem der vorherigen Ansprüchen,
**dadurch gekennzeichnet, dass**
Antigene, Liganden und/oder Antikörper zur Detektion des 4-1BB Rezeptors verwendet werden.

12. Verwendung nach einem der vorherigen Ansprüchen,
**dadurch gekennzeichnet, dass**
die Antigene, Liganden und/oder Antikörper zur Detektion des 4-1BB Rezeptors mit einem fluoreszierenden Stoff, Haptenen und/oder magnetischen Partikeln gekoppelt sind.

13. Verwendung nach einem der vorherigen Ansprüchen,
**dadurch gekennzeichnet, dass**
die regulatorischen Th-Zellen aus Blut, peripheren mononukleären Blutzellen (PBMC), Körpergewebe oder Zellen der Gewebeflüssigkeit identifiziert und/oder separiert werden.

14. Verwendung nach einem der vorherigen Ansprüche,
**dadurch gekennzeichnet, dass**
der 4-1BB Rezeptor Antikörper mit einem fluoreszierenden Stoff, Haptenen und/oder mit magnetischen Mikropartikeln gekoppelt ist.

15. Verwendung nach einem der vorherigen Ansprüche,
**dadurch gekennzeichnet, dass**
aktivierte regulatorische Th-Zellen identifiziert und/oder separiert werden.

16. Verwendung nach einem der vorherigen Ansprüche,
**dadurch gekennzeichnet, dass**
lebende regulatorische Th-Zellen identifiziert und/oder separiert werden.

17. Kit umfassend einen Antikörper zur Detektion des 4-1BB Rezeptors zur Identifikation und/oder Separation einer regulatorischen Th-Zelle.
